# EUROPÄISCHE PATENTSCHRIFT

(11) **EP 0 705 601 B1**
(45) Veröffentlichungstag und Bekanntmachung des Hinweises auf die Patenterteilung: **19.01.2000**
(21) Anmeldenummer: 94115597.0
(22) Anmeldetag: 04.10.1994
(51) Int. Cl.: A61K 31/12, A61K 31/19, A61K 31/70, A61K 47/14, A61K 47/22, A61K 47/40

(54) **Lösungen von Anthrachinonen zur parenteralen Applikation**
Anthraquinones containing solutions for parenteral administration
Solutions contenant des anthraquinones pour administration parentérale

(43) Veröffentlichungstag der Anmeldung: 10.04.1996
(73) Patentinhaber: LOMAPHARM Rudolf Lohmann GmbH KG Pharmazeutische Fabrik, 31860 Emmerthal (DE)
(72) Erfinder: Glänzer, Klaus Dr., D-22337 Hamburg (DE)
(74) Vertreter: Schwabe - Sandmair - Marx

(56) Entgegenhaltungen:
- EP-A- 0 154 117
- EP-A- 0 236 822
- EP-A- 0 256 285
- EP-A- 0 454 058
- WO-A-92/14454
- DATABASE WPI Week 9119, Derwent Publications Ltd., London, GB; AN 91-136894 (19) & JP-A-03 074 326 (TSUMURA & CO.) 28. März 1991
- DATABASE WPI Week 9029, Derwent Publications Ltd., London, GB; AN 90-220785 (29) & JP-A-02 149 515 (TSUMURA & CO.) 8. Juni 1990
- DATABASE WPI Week 8123, Derwent Publications Ltd., London, GB; AN 81-41287D (23) & JP-A-56 043 210 (HAYASHI K.) 21. April 1981
- DATABASE WPI Week 8925, Derwent Publications Ltd., London, GB; AN 89-182758 (25) & JP-A-01 121 215 (NIHON HAIPOKKUSU KK.) 12. Mai 1989

## Beschreibung

Die Erfindung betrifft stabile, hitzesterilisierbare Lösungen von Anthrachinonen (Anthrachinon und Anthrachinon-Derivate, einschließlich Anthrachinon-abspaltender Verbindungen, wie z.B. Glykoside), wie sie in den Ansprüchen definiert sind. Sie sind als pharmazeutische Zubereitungen für parenterale Anwendungen geeignet.

Anthrachinonverbindungen der genannten Art sind von großem Interesse, da sie nachgewiesenermaßen Hemmwirkungen gegenüber Viren und/oder Retroviren aufweisen. So konnte insbesondere gezeigt werden, daß Hydroxyderivate von Anthrachinonen zur Prophylaxe und Therapie von Erkrankungen geeignet sind, die von Viren der Familie Herpetoviridae (z.B. Herpes simplex-, Cytomegalie-, Varicella-Zoster-, Epstein-Barr-Virus) verursacht werden, siehe "Verwendung von Anthrachinonderivaten zur Prophylaxe und Therapie von Viruserkrankungen", May, G. et al., DE 40 13 023 C2 (1990) oder "Anthrachinones as a new class of antiviral agents against human immunodeficiency virus", Schinazi, R.F., et al., Antiviral Res. 13, 265 (1990).

Darüber hinaus wurden bei einigen dieser Verbindungen Anti-Tumor-Effekte gefunden, die möglicherweise auf eine Starkung des Immunsystems zurückzuführen sind, siehe "Studies on inhibitors of skin tumor promotion VI. Inhibitory effects on Epstein-Barr virus activation", Konoshima, T., et al., J. Nat. Prod. 52, 987 (1989). Neben antibakteriellen Eigenschaffen (May, G. et al., DE 40 13 023 C2 (1990); Schinazi, R.F. et al., Antiviral Res. 13, 265 (1990)) oder der Prophylaxe bei Harnsteinbildung ("Pharmacokinetic study of Alizarin in man", D. Lorenz, et al., Meth. and Find. Exptl. Clin. Pharmac 7, 637 (1985)) sind seit längerem phytotherapeutische Anwendungen oraler Zubereitungen mit anthrachinonhaltigen Pflanzenpräparaten bekannt, z.B. zur Therapie der Obstipation ("Monographien der Kommission E", Bundesanzeiger Nr. 133 vom 21.07.1993).

Auch wird berichtet, daß Extrakte aus anthrachinonbildenden Pilzen (z.B. mit flüssigem Polyethylenglykol, Glycerin, Glycol oder Glucosesirup) bzw. Extrakte aus Aloe vera (z.B. mit Glycerin, Ethylacetat, Aceton, Alkohol), mit Hemmwirkung gegenüber Viren und Retroviren, zu pharmazeutischen Zwecken geeignet seien ("Pharmazeutische Zubereitungen und ihre Verwendung", Roth, L. et al., DE 39 130 40 A1 (1989); "Aloe emodin and other anthraquinones and anthraquinone-like compounds from plants virucidal against herpes simplex virus", Sydiskis, R., und Owen, P.G., US Pat. 4,670,265 (1987)). In den zuletzt genannten Fällen wird die Wirksamkeit der Präparate auf die anthrachinonhaltigen Inhaltsstoffe zurückgeführt, ohne daß allerdings eine genau definierte Anthrachinonverbindung bzw. ein genau definiertes Gemisch derartiger Verbindungen vorliegt.

Die ungünstigen Lösungseigenschaften, speziell die geringe Wasserlöslichkeit der Mehrzahl der Anthrachinonderivate (Römpps Chemie-Lexikon, Franck'sche Verlagsbuchhandlung, Stuttgart; The Merck Index), insbesondere auch der therapeutisch interessanten Verbindungen, stellen ein Hindernis dar, sowohl hinsichtlich der Untersuchungen der spezifischen biologischen Aktivität als auch der Entwicklung und Anwendung als Arzneimittel. Von den in den früheren Publikationen (s.o.) genannten Extraktions- bzw. Lösungsmitteln ist allerdings keines in der Lage, die Löslichkeit von Anthrachinonen signifikant zu erhöhen und zugleich eine physiologische Unbedenklichkeit bezüglich parenteraler Applikation zu gewährleisten.

Es besteht deshalb ein prinzipielles Bedürfnis nach physiologisch verträglichen, parenteral verabreichbaren Lösungen von Anthrachinonen mit ausreichend hoher Konzentration bei schneller Bioverfügbarkeit der jeweiligen Verbindung, und zwar ohne die sonst bei peroraler Anwendung unvermeidlichen Resorptionseffekte. Bislang ist es jedoch noch nicht gelungen, ausreichend hohe Konzentrationen der Anthrachinone in für die parenterale Applikation geeigneten Lösungen zur Verfügung zu stellen.

Als grober Richtwert für eine therapeutisch wirksame Dosis im Falle einer einmaligen Injektion oder Infusion können auf Basis der einzigen bisher vorliegenden pharmakokinetischen Studie mit Alizarin ["Pharmacokinetic study of Alizarin in man", D. Lorenz, et al., Meth. and Find. Exptl. Clin. Pharmac. 7, 637 (1985)] 10 mg Anthrachinon angenommen werden, die dann bei einem Blutvolumen von 5 l (entsprechend etwa einem Körpergewicht von 70 kg) einen Anfangsblutspiegel von ca. 2 µg/ml ergeben. Bei Annahme einer Halbwertzeit von ca. 12 Std., einem Wert, der ebenfalls in der pharmakokinetischen Studie mit Alizarin ermittelt wurde (s.o. D. Lorenz, et al.), wäre somit ein Blutspiegel ≥ 0,5 µg/ml über einen Tag gegeben. Derartige Konzentrationen in einem Injektions- bzw. Infusionsvolumen von ≤ 10 ml können zwar nach bisherigen Kenntnissen in einigen organischen Lösungsmitteln (z.B. Ethylacetat, Aceton) erzielt werden, derartige Lösungen erfüllen jedoch nicht die Anforderungen für Parenteralia.

Dies gilt auch für Lösungen von Anthrachinonen, die hohe Konzentrationen an Polyethylenglykol, Glycerin, Alkohol oder Propylenglykol enthalten. Auch wenn mitunter genügend hohe Konzentrationen der Anthrachinone erreicht werden können, ist der Einsatz dieser Lösungen für parenterale Zwecke wegen der Gefahr unerwünschter Nebenwirkungen [z.B. Hämolyse, Schmerz, Thrombose, Thrombophlebitis ("Osmolalities of Propylene Glycol-Containing Drug Formulations for Parenteral Use", Doenicke, A., et al., Anesth. Analg. 75, 431 (1992); "Propylene Glycol 35% causes hemolysis in humans", Doenicke, A., et al., ibid., 78, S. 93 (1994)] sehr stark eingeengt.

Um diese Nebenwirkungen zu vermeiden, ist versucht worden, die Löslichkeit von Anthrachinonen in wäßrigen Lösungen zu erhöhen. Dazu werden üblicherweise Lösungsvermittler eingesetzt. Eine eigene Untersuchung des Anmelders mit den bekannten, handelsüblichen Substanzen Chremophor® (Polyoxyethylenglycoltriricinoleat, Arzneibuchname Polyoxyl 35 bzw. 40 Castor Oil USP/NF) oder Soluphor P® (2-Pyrrolidon) ergab, daß diese als Lösungsvermittler für Anthrachinone zwar geeignet, jedoch aufgrund ihres toxikologischen Profils bzw. Nebenwirkungsprofils allenfalls im veterinärmedizinischen Bereich einsetzbar sind. Ihre Anwendung für parenterale Humanarzneimittel ist dagegen zweifelhaft und nach heutigem Kenntnisstand nicht mehr vertretbar.

In weiteren Untersuchungen zeigte sich, daß Mikroemulsionen (O/W-Emulsionen mit Sojabohnenöl, Baumwollsamenölen, halbsynthetischen Neutralölen, z.B. Miglyol®) oder Liposomen (Phospholipide) keine geeigneten Trägersubstanzen für Anthrachinone darstellen: entweder wurden die erwünschten Konzentrationen nicht erreicht, oder die Stabilität der Zubereitungen erwies sich als unbefriedigend. Bei einigen Anthrachinonen (z.B. Danthron, Purpurin oder Hypericin, einem Anthrachinondimeren) ist eine Löslichkeitsverbesserung durch Alkalisieren möglich, die aber in der Regel unzulänglich ist, d.h. es werden lediglich therapeutisch unzureichende Konzentrationen erzielt, während die Alkalisierung physiologisch nicht vertretbare Alkalikonzentrationen erfordert bzw. die Stabilität der Lösungen ungünstig beeinflußt.

Es ist daher Aufgabe der Erfindung, die oben genannten Nachteile des Standes der Technik zu überwinden. Konkreter ist es Aufgabe der Erfindung, für die parenterale Applikation geeignete Lösungen von Anthrachinon und dessen Derivaten zur Verfügung zu stellen, welche den Wirkstoff in ausreichenden Mengen, d.h. entsprechenden Konzentrationen, enthalten, um therapeutisch relevante Blutspiegel zu erhalten.

Überraschenderweise wurde nun gefunden, daß die Löslichkeit der Anthrachinone durch den Einsatz von mindestens einem Lösungsvermittler ausgewählt aus
I. Polyethylenglykol-660-12-Hydroxystearat
II. Tetrahydrofurfurylalkoholpolyethylenglykolether
III. Wasserlösliche Beta- und Gamma-Cyclodextrinderivate,
allein oder in Kombination mit anderen, physiologisch geeigneten, nichtwäßrigen Lösungsmitteln bzw. Lösungsvermittlern um mehr als das Hundertfache ihrer Löslichkeit in rein wäßriger Phase erhöht werden kann.

Als zusätzliche Lösungsmittel in der Kombination kommen beispielhaft Polyoxyethylen-Polyoxypropylen-Blockpolymere ("Polaxamer USP/NF", Lutrol®-F-Typen), 2-Pyrrolidon (Soluphor® P), Ethanol, Benzylalkohol, Phenylethylalkohol, flüssige Polyethylenglykole (Lutrol®-E-Typen), Glycerin, Propylenglykol, Butylenglykol oder deren Mischungen in Betracht.

Während z.B. die Löslichkeit eines der bekanntesten Anthrachinone, dem Alizarin, in Wasser bei Raumtemperatur lediglich ca. 2 µmol/l (≈ 0,48 mg/l) beträgt (Römpps Chemie-Lexikon, Franck'sche Verlagsbuchhandlung, Stuttgart; The Merck Index), kann diese mit den erfindungsgemäßen Lösungsvermittlern um mehr als das Tausendfache auf > 2,4 g/l (> 10 mmol/l, siehe Beispiel 1), > 9,6 g/l (> 40 mmol/l, siehe Beispiel 2) bzw. > 0,72 g/l (> 3 mmol/l, siehe Beispiel 3) gesteigert werden.

Mit Hilfe der erfindungsgemäßen Lösungsvermittler lassen sich die Anthrachinone der Formel: 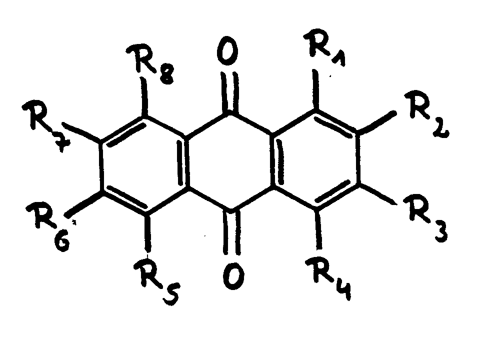 in der R₁ bis R₈, die gleich oder verschieden sein können und H, OH, C₁₋₄-Alkyl, NH₂ oder OR₉ bzw. NHR₁₀ bedeuten, wobei R₉ und R₁₀ C₁₋₄-Alkyl oder einen glycosidisch gebundenen Zuckerrest darstellen, sowie deren Dimere in wäßrige Lösungen mit den gewünschten Eigenschaften überführen. Diese Lösungen können zusätzlich die für die Herstellung pharmazeutischer Präparationen üblichen Hilfsstoffe enthalten.

Besonders bevorzugte Anthrachinone sind Alizarin, Anthraflavinsäure, Chrysazin, Chinizarin, Purpurin, Emodin, Chinalizarin, Rhein, Chrysophansäure, 1,4-Dihydroxy-2,3-dimethylanthrachinon, 1-Amino-4-hydroxyanthrachinon sowie Karminsäure, wobei die Reste R₁-R₈ wie in Tabelle 1 angegeben sind.

Als C₁₋₄-Alkylreste sind Methyl, Ethyl, n- sowie i-Propyl und n-, i- und t-Butyl zu nennen, wobei R₂ und R₃ bevorzugt Methyl darstellen.

Als Zuckerreste können alle üblichen Glycoside eingesetzt werden. Besonders bevorzugt sind Glucose, Mannose, Galactose, Arabinose, N-Acethylglucosamin, Muraminsäure, Neuraminsäure sowie die entsprechenden Alkohole und Polyglycane.

Die erfindungsgemäßen Lösungsvermittler sind ausgewählt aus
1. Polyethylenglykol-660-12-Hydroxystearat,
2. Tetrahydrofurfurylalkoholpolyethylenglykolether
3. Wasserlösliche β- und γ-Cyclodextrinderivate.

Polyethylenglykol-660-12-Hydrosystearat (z.B. Solutol® der BASF) ist ein relativ neuer nichtionogener Lösungsvermittler für Injektionszwecke (insbesondere für Vitaminpräparate sowie Miconazol- und Propanididzubereitungen), der bisher jedoch in keinem Arzneibuch beschrieben ist. Es existieren nach Firmeninformationen (Technische Information Solutol®, BASF) umfangreiche Unterlagen zu Toxizität und Nebenwirkungen, die insgesamt ein zufriedenstellendes toxikologisches Profil aufzeigen; ein Solutol® enthaltendes Humanarzneimittel befindet sich bereits im Handel.

Überraschend wurde gefunden, daß Anthrachinone in Solutol® HS 15 sehr gut löslich sind. Diese Lösungen können mit wäßriger Phase verdünnt werden und sind für die Herstellung von Lösungen von Anthrachinonen mit den eingangs definierten Eigenschaften geeignet. Die erfindungsgemäßen Lösungen enthalten zwischen 1 und 90 Gew.-%, vorzugsweise 10-60 Gew.-% und in noch bevorzugterer Weise 20 - 40 Gew.-% des Lösungsvermittlers.

Tetrahydrofurfylalkoholpolyethylenglykolether (TFPG) sind Ether mit einer oder mehreren Ethylenoxid-Einheiten und deren Gemische. Ein handelsüblicher TFPG ist z.B. unter den Bezeichnungen Glycofurol®, Glycofurol 75® und Tetraglykol® erhältlich. Vorzugsweise wird ein Produkt verwendet, das zu gleichen Teilen aus Tetrahydrofurfurylalkoholpolyethylenglykolether und Tetrahydrofurfurylalkoholpolydiethylenglykolether (CAS-Nr. 9004-76-6) besteht.

Die physiologische Unbedenklichkeit von TFPG (Tetrahydrofurfurylalkoholpolyethylenglykolether) bei parenteraler Applikation ist seit langem bekannt und belegt. Zahlreiche Injektions- und Infusionspräparate (Benzodiazepine, Phenytoin, Cotrimoxazol, Vitamine) mit diesem Lösungsvermittler sind als Arzneimittel zugelassen und im Handel. Überraschenderweise wurde gefunden, daß Anthrachinone in TFPG sehr gut löslich sind. Die erfindungsgemäßen Lösungen enthalten im allgemeinen 10 - 80 Vol.-% Tetrahydrofurfurylalkoholpolyethylenglykolether, vorzugsweise 20 - 70 Vol.-% und in besonders bevorzugter Weise 30 - 60 Vol.-%. Die Lösungen sind hitzesterilisierbar und über einen unerwartet langen Zeitraum stabil.

TFPG enthaltende Lösungen, besonders solche mit hohen Anthrachinonkonzentrationen, neigen jedoch in einigen Fällen bei Erhöhung des Wasseranteils, d.h. bei Verdünnung mit Wasser oder üblichen Infusionslösungen, spontan oder innerhalb kurzer Zeit zu Trübungen und Auskristallisationen. Mit derartigen Kristallabscheidungen verunreinigte Lösungen dürfen dann aus Gründen der Arzneimittelsicherheit nicht mehr parenteral verabreicht werden, da schwerwiegende Nebenwirkungen auftreten können.

Überraschenderweise wurde jedoch gefunden, daß in solchen Fällen ein Auskristallisieren unterbunden oder zumindest zeitlich erheblich verzögert (um mehr als 12 Stunden) werden kann, wenn den Lösungen Polyvinylpyrrolidon als Stabilisator zugegeben wird. Polyvinylpyrrolidon ist als Arzneimittelhilfsstoff in Arzneibüchern beschrieben und in unterschiedlichen Typen im Handel erhältlich. Erfindungsgemäß bevorzugt sind Polyvinylpyrrolidone mit einem Molekulargewicht bis etwa 40.000, entsprechend einem K-Wert im Bereich von 10-30. Für die parenterale Anwendung besonders geeignet sind PVP-Typen mit einem K-Wert im Bereich von 10-20. Derartig stabiliserte Lösungen sind dann vorteilhaft, wenn parenterale Lösungen mit dem Lösungsvermittler TFPG als Infusionszusatz gewünscht werden. Die erfindungsgemäßen Lösungen mit Tetrahydrofurfurylalkoholpolyethylenglykolether als Lösungsvermittler und zusätzlich Polyvinylpyrrolidon als Stabilisator enthalten im allgemeinen 10 - 80 Gew.-% TFPG und 1 - 10 Gew.-% Polyvinylpyrrolidon. Vorzugsweise werden 20 - 70 Gew.-% TFPG und 1 - 5 Gew.-% PVP und noch stärker bevorzugt 30 - 60 Gew.-% TFPG und 3 - 5 Gew.-% PVP eingesetzt.

Cyclodextrinderivate und ihre spezifischen Eigenschaften als Löslichkeitsvermittler sind bekannt und in der Literatur in Übersichtsartikeln dargestellt und diskutiert. Für parenterale Zwecke werden wasserlösliche veretherte β- bzw. γ-Cyclodextrin-Verbindungen bevorzugt. Im Falle der intravenösen Injektion oder Infusion können sich in seltenen Fällen Nebenwirkungen ergeben. Die geringsten Nebenwirkungen weist nach derzeit vorliegenden Untersuchungen das Hydroxypropyl-β-Cyclodextrin auf. Es ist daher bevorzugt.

Überraschenderweise wurde gefunden, daß Anthrachinone auch mit Cyclodextrin als Lösungsvermittler in wäßrigen Lösungen überführt werden können. Dabei wurde festgestellt, daß in den erfindungsgemäßen Lösungen das molare Verhältnis von gelöstem Anthrachinon und gelöstem Cyclodextrin annähernd 1:1 beträgt; d.h., mit 1 Mol Cyclodextrin als Lösungsvermittler kann 1 Mol Anthrachinon in Lösung gebracht werden. Die UV-VIS-spektroskopische Untersuchung dieser Lösungen zeigte keine signifikanten Abweichungen von Spektren, die mit Lösungen der entsprechenden Anthrachinone bei gleichem pH-Wert in anderen Solventien erhalten wurden.

Eine Kombination mit anderen, bereits genannten Lösungsmitteln bzw. Lösungsvermittlern ist möglich und in Einzelfällen sinnvoll, z.B. wenn damit die Löslichkeit von Cyclodextrin verbessert und gleichzeitig die Löslichkeit der Anthrachinone erhöht und die Lösung stabilisiert wird. Die erfindungsgemäßen Lösungen enthalten 5 - 40 Gew.-%, vorzugsweise 10 - 25 Gew.-% und am meisten bevorzugt 15 Gew.-% eines oder mehrerer Cyclodextrine.

Die erfindungsgemäßen Zubereitungen enthalten Anthrachinon oder dessen Derivate, wie in Anspruch 1 definiert, in Konzentrationen von > 10⁻⁵ mol/l, bevorzugt von > 10⁻⁴ mol/l und noch stärker bevorzugt von > 10⁻³ mol/l.

Neben den erfindungsgemäßen Lösungsvermittlern können auch weitere, nichtwäßrige Lösungsvermittler oder Lösungsmittel, wie Polyoxyethylen-Polyoxypropylen-Blockpolymere ("Polaxamer USP/NF", Lutrol®-F-Typen), 2-Pyrrolidon (Soluphor® P), Ethanol, Benzylalkohol, Phenylethylalkohol, flüssige Polyethylenglykole (Lutrol®-E-Typen), Glycerin, Propylenglykol, Butylenglykol oder deren Mischungen, wie in den nachfolgenden Beispielen aufgeführt, eingesetzt werden, um Anthrachinone in Lösungen mit den gewünschten Eigenschaften zu überführen. Wegen der bereits erwähnten, unerwünschten Nebenwirkungen ist in den erfindungsgemäßen Lösungen der Einsatz dieser zuletzt genannten, zusätzlichen Lösungsvermittler eingeschränkt. Im Hinblick aufbestmögliche physiologische Verträglichkeit der erfindungsgemäßen Lösungen bei der klinischen Anwendung werden sie als Zusatzkomponente je Substanz im allgemeinen in einer Menge von max. 40 Vol.-%, bevorzugt bis zu 20 Vol.-% eingesetzt. Besonders bevorzugt beträgt ihr Mengenanteil bis zu 5 Vol.-%.

Um bestimmte Eigenschaften hinsichtlich Viskosität, Osmolalität, Stabilität, Pufferwirkung oder andere pharmazeutisch-technische Eigenschaften der erfindungsgemäßen Lösungen zu erzielen, sind neben den genannten Lösungsmitteln und Lösungsvermittlern weitere Zusätze wie Polyvinylpyrrolidon (s. insbesondere Beispiel 2), Puffersysteme (z.B. Phosphatpuffer, Citratpuffer, Acetatpuffer), Stoffe zur Isotonisierung (z.B. Salze wie Natriumchlorid), an Konservierungsmitteln und Komplexbildnern (Edetinsäure bzw. ihre Salze) als Bestandteile in den üblichen Mengen zu nennen. Zur näheren Erläuterung der Erfindung sollen die nachfolgenden Beispiele dienen, in denen die in Tabelle 1 aufgeführten Anthrachinone exemplarisch eingesetzt wurden.

### Beispiel 1

### Herstellung parenteraler Zubereitungen mit Solutol® HS 15

Handelsübliche, chemisch reine Anthrachinone wurden durch Umkristallisieren aus Ethanol, Isopropanol oder Mischungen beider Lösungsmittel gereinigt, getrocknet und einer chemischen und physikalischen Untersuchung unterzogen (z.B. Prüfung auf Identität mittels IR- und UV-VIS-Spektroskopie, Reinheit mittels DC, Schmelzpunkt).

Die Substanzen wurden in Solutol® HS 15 bei ca. 40-60 Grad Celsius gelöst und in die auf ca. 50-65 °C erwähnte wäßrige Phase langsam eingearbeitet. Die wäßrige Phase enthielt entweder keine, einzelne oder mehrere, d.h. Kombinationen, der oben aufgeführten Formulierungshilfen (weitere Lösungsmittel bzw. Lösungsvermittler, Puffersysteme, Stabilisatoren usw.), je nach gewünschter Konzentration und Art des zu lösenden Anthrachinonderivates sowie der gewünschten Eigenschaften hinsichtlich chemischer und physikalischer Stabilität bzw. anderer pharmazeutischer Anforderungen. Die Lösungen wurden sterilfiltriert, in Injektionsfläschchen oder Ampullen abgefüllt und hitzesterilisiert.

In Tabelle 2 sind beispielhaft erfindungsgemäße Formulierungen zusammengestellt, die den eingangs definierten Anforderungen an parenteral applizierbare Lösungen von Anthrachinonen entsprechen.

### Beispiel 2

### Herstellung parenteraler Zubereitungen mit Tetrahydrofurfurylalkoholpolyethylenglykolether

Durch Umkristallisieren gereinigte Anthrachinone (so.) wurden bei Raumtemperatur in der gewünschten Menge Glykofurol 75® als Tetrahydrofurfurylalkoholpolyethylenglykolether gelöst und mit der wäßrigen Phase vermischt. Die wäßrige Phase enthielt entweder keine, einzelne oder mehrere, d.h. Kombinationen, der oben aufgeführten Formulierungshilfen (Polyvinylpyrrolidon als Stabilisator für die zum Verdünnen mit wäßrigen Phasen besonders geeigneten Zubereitungen, Puffersysteme, weitere Lösungsvermittler, Stabilisatoren usw.), je nach gewünschter Konzentration und Art des zu lösenden Anthrachinonderivats, sowie der gewünschten Eigenschaften hinsichtlich chemischer und physikalischer Stabilität sowie anderer pharmazeutischer Anforderungen.

Die Lösungen wurden sterilfiltriert, in Injektionsfläschchen oder Ampullen abgefüllt und hitzesterilisiert. In Tabelle 3 sind beispielhafte erfindungsgemäße Formulierungen zusammengestellt, die den eingangs definierten Anforderungen an parenteral anwendbare Lösungen von Anthrachinonderivaten entsprechen.

### Beispiel 3

### Herstellung parenteraler Zubereitungen mit Hydroxypropyl-β-Cyclodextrin

Durch Umkristallisieren gereinigte Anthrachinone (s.o.) wurden unter Rühren in eine auf ca. 40 Grad Celsius erwähnte Lösung der Cyclodextrinverbindung gegeben. Nach vollständiger Auflösung wurde sterilfiltriert, in Injektionsfläschchen oder Ampullen abgefüllt und hitzesterilisiert. Gleichermaßen können auch Trockenmischungen aus Cyclodextrin und Anthrachinon oder durch Eindampfen oder als Lyophilisat aus Cyclodextrin-Anthrachinon-Lösungen erhaltene Trockenmischungen mit einem handelsüblichen Injektions- bzw. Infusionspräparat (Wasser für Injektion, physiologische Kochsalzlösung, usw.) zu einer injizierbaren Zubereitung verarbeitet werden.

In Tabelle 4 sind beispielhaft erfindungsgemäße wäßrige Formulierungen zusammengestellt, die den eingangs definierten Anforderungen an parenteral anwendbare Lösungen von Anthrachinonen entsprechen.

**Tabelle 1**

| Übersicht der untersuchten Anthrachinone | | | | | | | | |
|---|---|---|---|---|---|---|---|---|
| Strukturformel: | | | | | | | | |

| Verbindung | 1 | 2 | 3 | 4 | 5 | 6 | 7 | 8 |
|---|---|---|---|---|---|---|---|---|
| (1) Alzarin | OH | OH | --- | --- | --- | --- | --- | --- |
| (2) Anthraflavinsäure | --- | OH | --- | --- | --- | OH | --- | --- |
| (3) Chrysazin (= Danthron) | OH | --- | --- | --- | --- | --- | --- | OH |
| (4) Chinizarin | OH | --- | --- | OH | --- | --- | --- | --- |
| (5) Purpurin | OH | OH | --- | OH | --- | --- | --- | --- |
| (6) Emodin | OH | --- | OH | --- | --- | OH | --- | OH |
| (7) Chinalizarin | OH | OH | --- | --- | OH | --- | --- | OH |
| (8) Rhein | --- | COOH | --- | OH | OH | --- | --- | --- |
| (9) Chrysophansäure | OH | --- | CH₃ | --- | --- | --- | --- | OH |
| (10) 1.4-Dihydroxy-2.3-diemthylanthrachinon | OH | CH₃ | CH₃ | OH | --- | --- | --- | --- |
| (11) 1-Amino-4-hydroxyanthrachinon | NH₂ | --- | --- | OH | --- | --- | --- | --- |
| (12) Karminsäure (R=Glucose) | CH₃ | COOH | OH | --- | OH | OH | R | OH |

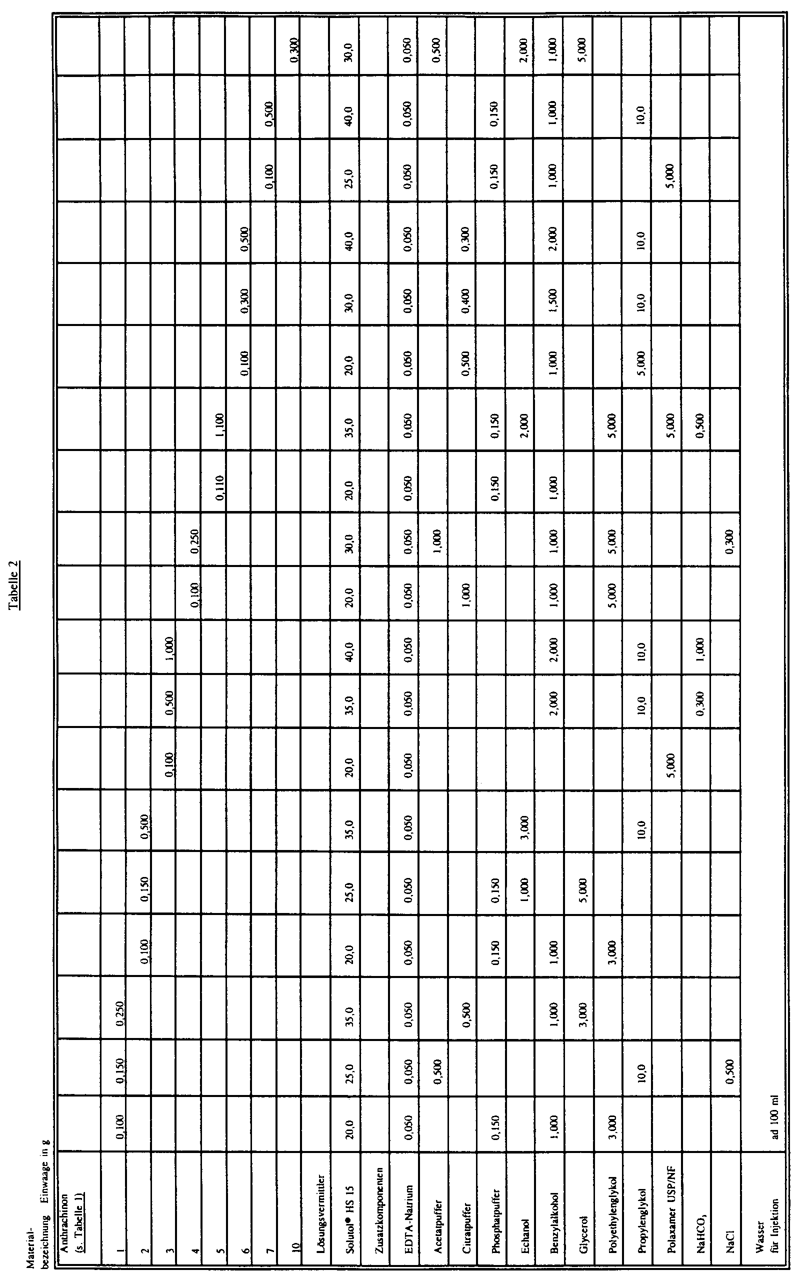

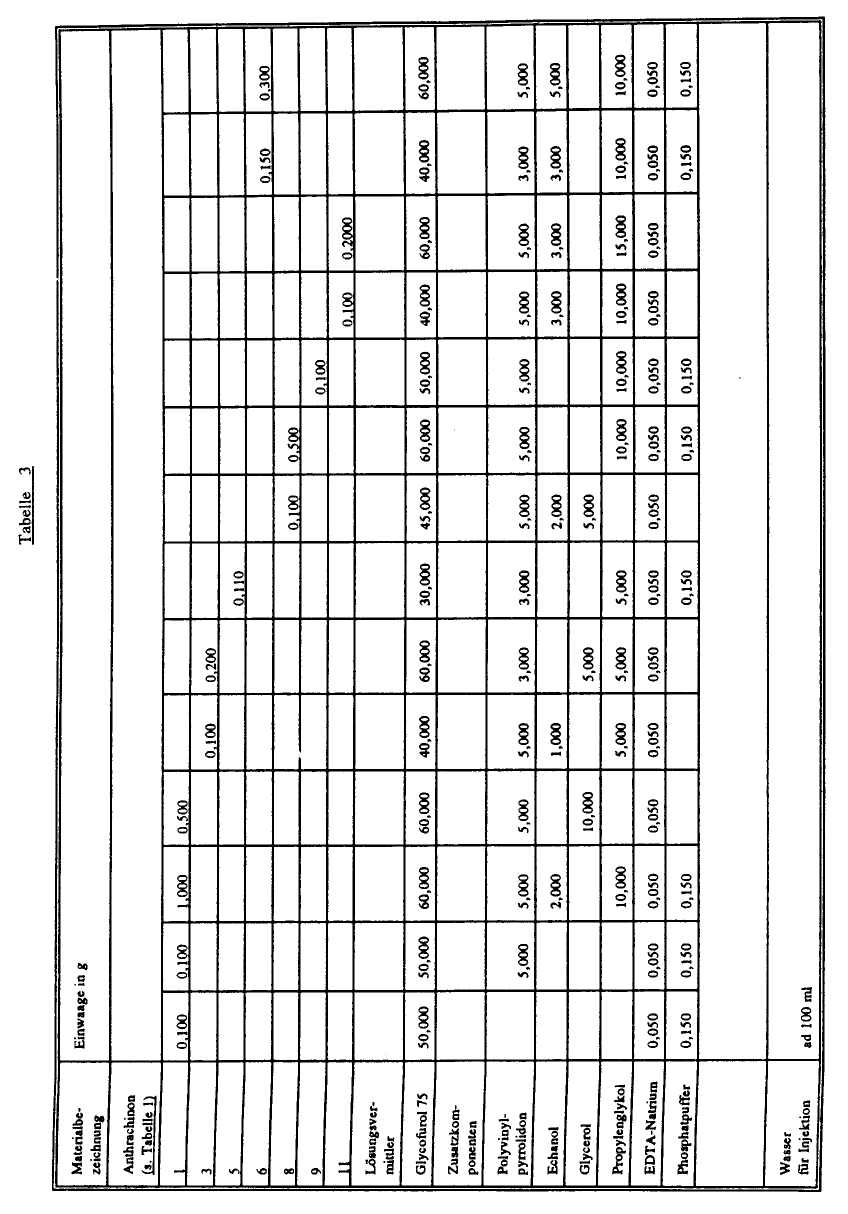

**Tabelle 4**

| Materialbezeichnung | Einwaage in g | | | | | | | | |
|---|---|---|---|---|---|---|---|---|---|
| Anthrachinon (s.Tab. 1) | | | | | | | | | |
| 1 | 0,010 | 0,020 | 0,075 | | | | | | |
| 2 | | | | | | 0,020 | | | |
| 3 | | | | | | | 0,020 | | |
| 4 | | | | | | | | 0,020 | |
| 5 | | | | | | | | | 0,025 |
| 8 | | | | | 0,030 | | | | |
| 12 | | | | 0,300 | | | | | |

| Lösungsvermittler | | | | | | | | | |
|---|---|---|---|---|---|---|---|---|---|
| Hydroxypropyl-β-Cyclodextrin | 10,000 | 15,000 | 25,000 | 25,000 | 20,000 | 15,000 | 15,000 15,000 | | 15,000 |

| Zusatzkomponenten | | | | | | | | | |
|---|---|---|---|---|---|---|---|---|---|
| Ethanol | 1,000 | | | | | | | | |
| Glycerol | | | | 2,000 | | | | | |
| Polyethylenglykol | | | 10,000 | | | | | | |
| Phosphatpuffer | 0,150 | 0,150 | 0,150 | 0,150 | 0,150 | 0,150 | 0,150 | 0,150 | 0,150 |
| EDTA-Natrium | 0,050 | 0,050 | 0,050 | 0,050 | 0,050 | 0,050 | 0,050 | 0,050 | 0,050 |
| | | | | | | | | | |

| Wasser für Injektion | ad 100 ml | | | | | | | | |
|---|---|---|---|---|---|---|---|---|---|
| | | | | | | | | | |

## Patentansprüche

1. Stabile, hitzesterilisierbare Lösungen von Anthrachinonen, welche ein oder mehrere Anthrachinone der Formel 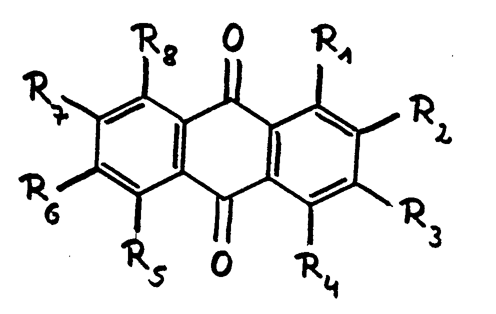 in der R₁ bis R₈, die gleich oder verschieden sein können, H, OH, C₁₋₄-Alkyl, NH₂ oder OR₉ bzw. NHR₁₀ bedeuten, wobei R₉ und R₁₀ C₁₋₄-Alkyl oder einen glykosidisch gebundenen Zuckerrest darstellen, sowie deren Dimere oder Rhein und Karminsäure in einer Konzentration von > 10⁻⁵mol/l zusammen mit mindestens einem Lösungsvermittler aus
I. Polyethylenglykol-660-12-Hydroxystearat
II. Tetrahydrofurfurylalkoholpolyethylenglykolether
III. Wasserlösliche β- und γ-Cyclodextrinderivate,
allein oder in Kombination mit einem oder mehreren anderen, physiologisch geeigneten, nichtwässrigen Lösungsmitteln bzw. Lösungsvermittlern sowie Wasser und ggf. zusätzliche für die Herstellung von pharmazeutischen Zubereitungen übliche Hilfsstoffe enthalten.

2. Lösung gemäß Anspruch 1, dadurch gekennzeichnet, daß das Anthrachinonderivat
Alizarin,
Anthraflavinsäure,
Chrysazin,
Chinizarin,
Purpurin,
Emodin,
Chinalizarin,
Chrysophansäure,
1,4-Dihydroxy-2,3-dimethylanthrachinon oder
1-Amino-4-hydroxyanthrachinon ist.

3. Lösung gemäß Anspruch 1 oder 2, dadurch **gekennzeichnet**, daß sie die Anthrachinone in einer Konzentration > 10⁻³ mol/l enthält.

4. Lösung gemaß einem der Ansprüche 1 bis 3, dadurch **gekennzeichnet,** daß sie als weitere Lösungsvermittler Polyoxyethylen-Polyoxypropylen-Blockpolymere, 2-Pyrrolidon, Ethanol, Benzylalkohol, Phenylethylalkohol, flüssige Polyethylenglykole, Glycerin, Propylenglykol, Butylenglykol oder deren Mischungen enthält.

5. Lösung gemaß einem der vorstehenden Ansprüche, dadurch **gekennzeichnet,** daß sie Polyethylenglykol-660-12-Hydroxystearat in einer Menge von 1-90 Gew.-%, vorzugsweise 10-60 Gew.-%, in noch bevorzugterer Weise 20-40 Gew.-% enthält.

6. Lösung gemaß einem der Ansprüche 1 bis 4, dadurch **gekennzeichnet,** daß sie Tetrahydrofurfurylalkoholpolyethylenglykolether (TFPG) in einer Menge von 10 - 80 Gew.-%, vorzugsweise 20 - 70 Gew.-% und besonders bevorzugt 30 - 60 Gew.-% enthält.

7. Lösung gemäß Anspruch 6, dadurch **gekennzeichnet**, daß sie in Kombination mit TFPG Polyvinylpyrrolidon in einer Menge von 1-10 Gew.-%, vorzugsweise 1-5 Gew.-% und besonders bevorzugt von 3-5 Gew.-% enthält.

8. Lösung gemäß einem der Ansprüche 1 bis 4, dadurch **gekennzeichnet**, daß sie die Cyclodextrinderivate als Lösungsvermittler in einer Menge von 5-40 Gew.-%, vorzugsweise 10-25 Gew.-% und am meisten bevorzugt 15 Gew.-% enthält.

## Claims

1. Stable, heat-sterilisable anthraquinone solutions containing one or more anthraquinones corresponding to the formula 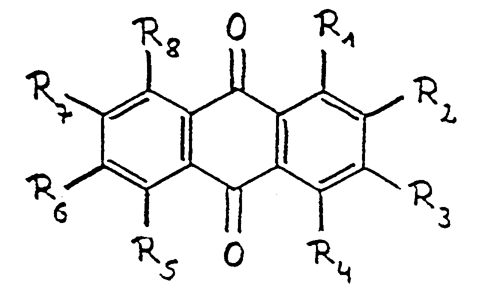 in which R₁ to R₈ are the same or different and denote H, OH, C₁₋₄-alkyl, NH₂ or OR₉ or NHR₁₀, with R₉ and R₁₀ representing C₁₋₄-alkyl or a glycosidically bound sugar group, as well as dimers thereof or rheinic and carminic acid at a concentration of > 10⁻⁵ mol/l together with at least one solubility promoter comprising
I. polyethylene glycol-660-12-hydroxystearate
II. tetrahydrofurfuryl alcohol polyethylene glycol ether
III. water-soluble β- and γ-cyclodextrin derivatives,
alone or in combination with one or more other physiologically suitable non-aqueous solvents or solubility promoters, as well as water and optionally additional auxiliary substances conventionally used in the preparation of pharmaceutical preparations.

2. Solution according to Claim 1, characterised in that the anthraquinone derivative is
alizarin
anthraflavic acid
chrysazin
quinizarin
purpurin
emodin
quinalizarin
chrysophanic acid
1,4-dihydroxy-2,3-dimethylanthraquinone or
1-amino-4-hydroxyanthraquinone.

3. Solution according to Claim 1 or 2, characterised in that it contains anthraquinones at a concentration of > 10⁻³ mol/l.

4. Solution according to one of Claims 1 to 3, characterised in that it contains as further solubility promoters polyoxyethylene-polyoxypropylene block polymers, 2-pyrrolidone, ethanol, benzyl alcohol, phenethyl alcohol, liquid polyethylene glycols, glycerol, propylene glycol, butylene glycol or mixtures thereof.

5. Solution according to one of the preceding claims, characterised in that it contains polyethylene glycol-660-12-hydroxystearate in a quantity of from 1 to 90 wt-%, preferably 10 to 60 wt-%, more preferably 20 to 40 wt-%.

6. Solution according to one of Claims 1 to 4, characterised in that it contains tetrahydrofurfuryl alcohol polyethylene glycol ether (TFPG) in a quantity of from 10 to 80 wt-%, preferably 20 to 70 wt-%, and particularly preferably 30 to 60 wt-%.

7. Solution according to Claim 6, characterised in that it contains polyvinyl pyrrolidone in a quantity of from 1 to 10 wt-%, preferably 1 to 5 wt-%, and particularly preferably 3 to 5 wt-%, in combination with TFPG.

8. Solution according to one of Claims 1 to 4, characterised in that it contains the cyclodextrin derivatives as solubility promoters in a quantity of from 5 to 40 wt-%, preferably 10 to 25 wt-%, and most preferably 15 wt-%.

## Revendications

1. Solutions stables d'anthraquinones, thermiquement stérilisables, qui contiennent une ou plusieurs anthraquinones de formule : 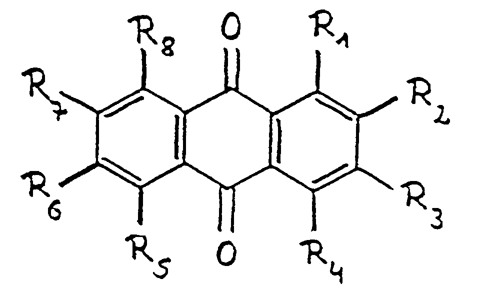 dans laquelle R₁ à R₈, qui peuvent être identiques ou différents, représentent H, OH, un groupe alkyle en C₁₋₄, NH₂ ou OR₉ ou NHR₁₀, R₉ et R₁₀ représentant un groupe alkyle en C₁₋₄ ou un reste de sucre à liaison glycosidique, ainsi que leurs dimères ou de l'acide rhéique et de l'acide carminique, en une concentration > 10⁻⁵ mole/litre, ainsi qu'un au moins un solubilisant choisi parmi
I. le 12-hydroxystéarate de polyéthylèneglycol 660
II. le polyéthylèneglycol éther d'alcool tétrahydrofurfurylique
III. les dérivés hydrosolubles des β- et γ-cyclodextrines,
soit seul soit en combinaison avec un ou plusieurs autres solvants ou solubilisants non aqueux, physiologiquement acceptables, ainsi que de l'eau et éventuellement, des adjuvants usuels dans la fabrication de préparations pharmaceutiques.

2. Solutions selon la revendication 1, caractérisées par le fait que les dérivés d'anthraquinone sont
l'alizarine,
l'acide anthraflavinique,
la chrysazine,
la quinizarine,
la purpurine,
l'émodine,
la quinalizarine,
l'acide chrysophanique,
la 1,4-dihydroxy-2,3-diméthylanthraquinone ou
la 1-amino-4-hydroxyanthraquinone.

3. Solutions selon la revendication 1 ou 2, caractérisées par le fait qu'elles contiennent les anthraquinones en une concentration >10⁻³ mole/litre.

4. Solutions selon l'une quelconque des revendications 1 à 3, caractérisées par le fait qu'elles contiennent, en tant qu'autre solubilisant, un copolymère séquencé polyoxyéthylène-polyoxypropylène, la 2-pyrrolidone, l'éthanol, l'alcool benzylique, l'alcool phényléthylique, un polyéthylèneglycol liquide, la glycérine, le propylèneglycol, le butylèneglycol ou des mélanges de ceux-ci.

5. Solutions selon l'une quelconque des revendications précédentes, caractérisées par le fait qu'elles contiennent le 12-hydroxy-stéarate de polyéthylèneglycol 660 en une proportion de 1-90 % en poids, de préférence de 10-60 % en poids, de préférence encore, de 20-40 % en poids.

6. Solutions selon l'une quelconque des revendications 1 à 4, caractérisées par le fait qu'elles contiennent le polyéthylène glycol éther d'alcool tétrahydrofurfurylique (TFPG) en une proportion de 10-80 % en poids, de préférence de 20-70 % en poids, et de façon particulièrement préférée, de 30-60 % en poids.

7. Solutions selon la revendication 6, caractérisées par le fait qu'elles contiennent, en combinaison avec le TFPG, de la polyvinylpyrrolidone en une proportion de 1-10 % en poids, de préférence de 1-5 % en poids, et de façon particulièrement préférée, de 3-5 % en poids.

8. Solutions selon l'une quelconque des revendications 1 à 4, caractérisées par le fait qu'elles contiennent le dérivé de cyclodextrine, en tant que solubilisant, en une proportion de 5-40 % en poids, de préférence de 10-25 % en poids, et de façon particulièrement préférée, de 15% en poids.
